# EUROPEAN PATENT APPLICATION

(11) **EP 1 970 056 A1**
(43) Date of publication of application: **17.09.2008**
(21) Application number: 07104213.9
(22) Date of filing: 15.03.2007
(51) Int. Cl.: A61K 9/24, A61K 9/28, A61K 31/519

(54) **Time-specific delayed/pulsatile release dosage forms**

(71) Applicant: POLICHEM S.A., 1526 Luxembourg (LU)
(72) Inventor: Cerea, Matteo, 24064 Grumello del Monte BG (IT); De Luigi Bruschi, Stefano, 20146 Milano (IT); Sangalli, Maria, Edvige, 27100 Pavia (IT); Zema, Lucia, 22100 Como (IT); Gazzaniga, Andrea, 27046 Santa Giuletta Pavia (IT)
(74) Representative: Pistolesi, Roberto

(57) **Abstract**

A time - specific delayed/pulsatile release dosage form which comprises:
**·** a core comprising at least one active principle and at least one disintegrating agent;
**·** a sealing layer surrounding the core essentially consisting of one or more water soluble or water insoluble pH independent polymers;
**·** an outer coating essentially consisting of one or more hydrophilic pH independent polymers;

wherein:
the at least one disintegrating agent is present in amounts of 1-20% by weight and
the at least one active principle is present in amounts of 1-80% by weight, with respect to the core;
the sealing layer accounts for 0.1-10% by weight, with respect to the core;
the outer coating accounts for 5-500 % by weight, with respect to the core.

These coated cores are capable of ensuring the immediate release of the active principle after a pre-defined lag time, independently of physiological pH variations occurring in the gastro-intestinal tract of mammals.

## Description

The present invention relates to pharmaceutical formulations for treating morning pathologies. Examples of specific late nigh-time or early morning pathologies include but are not limited to asthma, angina, hypertension, myocardial or cerebral infarction, arthritis, incontinence, sleep disorders, Parkinson's disease. The pharmaceutically active agent, delivered according to the delayed/pulsatile release formulations of the present invention, is an active principle effective against the condition or pathology being treated.

In particular, the invention relates to a time - specific delayed/pulsatile release dosage form which comprises:
- a core comprising at least one active principle and at least one disintegrating agent;
- a sealing layer surrounding the core essentially consisting of one or more water soluble or water insoluble pH independent polymers;
- an outer coating essentially consisting of one or more hydrophilic pH independent polymers;
wherein:
the at least one disintegrating agent is present in amounts of 1-20% by weight and
the at least one active principle is present in amounts of 1-80% by weight, with respect to the core;
the sealing layer accounts for 0.1-10% by weight, with respect to the core;
the outer coating accounts for 5-500 % by weight, with respect to the core.

These coated cores are capable of ensuring the immediate release of the active principle after a pre-defined lag time, independently of physiological pH variations occurring in the gastro-intestinal tract of mammals.

### STATE OF THE ART

By time-specific delayed/pulsatile -release formulation is meant a drug delivery system wherein release of the active agent (or agents) from the dosage form is modified to occur at a later time than that from a conventional immediate release product. The subsequent release of the pharmaceutical active ingredient from such a delayed/pulsatile release formulation is designed to release the drug agent so that it is promptly available for absorption at the release site after a predetermined period of time from the intake. Alternatively, the system can be designed to delay the delivery of a fraction of the active substance so that it is promptly available for absorption at the release site, and another fraction is released with a prolonged release kinetic after a predetermined period of time from the intake has passed. Examples of specific pharmaceutically active agents which may be included in the pharmaceutical formulations of the present invention include but are not limited to antiasthmatics, antihypertensive agents, anticoagulants, antiplatelet agents, anti-parkinsonian agents, sedatives, anxiolytic agents, anticholinergic and antispasmodic agents, vasopressin analogues, peptide and biopolymeric agents.

Delayed/pulsatile release systems are a relatively new class of modified drug delivery devices which represent a topic of interest.

The achievement of an optimal drug delivery pattern may require an integrated technology and physiological / pharmacokinetic approach which often relies on the concomitant control of both rate and time of release. In particular per-oral temporally modulated drug delivery systems have become a significant field of research since, besides including the principles of pulsatile release, they are considered useful in providing site-specific drug delivery in particular regions of the G.I. tract. In this respect particularly interesting are the colon-targeted drug delivery systems which employ the relative constancy of small intestine transit time for timing the colon drug release after a programmed lag-phase that prevents the release of the drug.

The role and influence of temporal rhythms (e. g. circadian) on many biological processes and pathologies are well known as the consequent needs for therapies reflecting the time pattern of such rhythms. Examples of pathologies requiring this sort of approach may include ischemic heart diseases, asthma, arthritis, sleep disorder and pain. On the other hand there is an increasing interest in delivering drugs to a specific site in the GI tract for example to face therapeutics needs such as bowel inflammatory diseases or to improve oral bioavailability of drugs by targeting a more favourable absorption window. This is the case of peptides and proteic drugs which encounter a less aggressive environment in the colonic region due to the low concentration of enzymatic activity. Therefore, a strong rationale is generally accepted for the development of systems capable of releasing drugs at predetermined times and /or sites following oral administration. (Sangalli M.E. et al. In vitro and in vivo evaluation of an oral system for time and/or site-specific drug delivery, Journal of Controlled Release 2001 73:1 (103-110))

The delivery system object of the present invention consists on a rapidly releasing core which is coated with one or more hydrophilic polymers and is designed to give a programmed silent phase before the onset of drug release. Drug release is designed to be time dependent and pH independent to ensure a high degree of site specificity.

The formulation design of the coated dosage units should ensure that the rapidly releasing core preserves its original drug release pattern after the programmed lag phase which lasts the time needed by the physiological aqueous fluids to erode and /or dissolve the external polymeric layer.

These systems are recommended for therapies requiring effective blood levels few hours after oral administration e.g. at specific night periods or before the patient awakening. Moreover, through proper modifications of the design and controlling the lag phase duration, delivery to a specific site in the gastrointestinal tract (e.g.) colon can be achieved. (Gazzaniga A. et al. Time-controlled oral delivery systems for colon targeting, Expert Opinion on Drug Delivery 2006 3:5 (583-597))

Actually, most available systems consist of drug-containing cores which are coated with polymers having pH - dependent characteristics. These polymers, depending on their chemical nature, are designed to be insoluble at pH below 6 to 7 and soluble at higher pHs in order to exploit a hypothetical constant increase of the pH from the stomach to the large intestine. The coating of the units is therefore assumed to retain its integrity thus preventing the release of the active during the transit through the stomach and small intestine and then to dissolve on arrival at the colon. However, the pH profile along the human gastrointestinal tract does not follow the supposed gradient.

Therefore, for coatings that dissolve at pH above 7, there is a reasonable risk that the dosage form may start releasing in the ileum rather than in the colon or even more problematic if remains intact in the small intestine. As a matter of fact, it has been demonstrated that the pH values of the intraluminal colon environment in patients with inflammatory bowel disease (IBD) is significantly decreased when compared to healthy volunteers compromising the triggering mechanism of the protective film (Fallingborg, J et al. Very low intraluminal colonic pH in patients with active ulcerative colitis. Dig Dis Sci, 38 (1993) 89-93). There is uncertainty deriving from the pH environment about the location in which pH dependent formulations can start to release bioactive agents. To overcome the limitations of the delayed/pulsatile release systems based on pH dependent polymers, alternative coating system based on hydrophilic pH independent polymers were developed. The delay is based on the slow interaction with aqueous fluids of the hydrophilic polymer layer applied onto a drug containing core.

The hydrophilic coating can be applied using known techniques such as spray coating and powder layering. When spray coating is used, the polymer/s is solubilized in aqueous solution which is sprayed onto the cores by means of any suitable coating apparatus including but not limited to fluid bed or coating pan.

Alternatively, the polymeric coating layer is applied onto the cores by power layering. This technique relies on the layering process of powdered-polymeric coating mixtures onto solid drug containing cores by continuously or alternatively spraying a liquid binder. During the process the core surface is sprayed with the binder solution which promotes the adherence of the powdered-polymeric coatings onto the core. Suitable liquid binder may include conventional pharmaceutically acceptable binding agents such as solutions of polymeric matters in appropriate solvents.

When the units enter in contact with physiological fluids, the hydrophilic layer starts to swell. The slow interaction between polymer and aqueous medium leads to the formation of a gel through a glassy rubbery transition with consequent thickness increase. The gel layer, depending on both the characteristics of polymeric components and composition, progressively erodes and /or becomes freely permeable. This determines the duration of the lag phase as a function of the original layer thickness. The interaction between solvent and polymer can be followed through the movements of the eroding and swelling fronts. In this respect, their rapid synchronization, along with the consequent minimal thickness of the gel layer, represents, as far as drug release pattern is concerned, the main requirement to achieve the desired burst or pulse effect.

The duration of the lag phase of the of the drug release can be tuned by means of the modulation of the thickness of the hydrophilic pH independent polymers layered onto the cores.

Unfortunately, the pH independent hydrophilic polymers may interact with the core, creating a matrix effect which slows down the drug dissolution. Since as for many drugs that need to be delivered according to the circadian cycles, it is often desirable that release occurs immediately once the programmed lag time is elapsed, it is essential to formulate coated cores still possessing the same disintegrating properties of those uncoated. Hence, to reach adequate peak plasma levels, a desirable property of time independent and pH independent delayed drug delivery systems is the immediate drug release once the external hydrophilic layer is totally or partially eroded.

US 5,788,987 teaches a method for the manufacturing of delayed release tablets made by fast disintegrating cores that are successively coated with hydrophilic pH independent polymers based mainly on ethers of cellulose.

The fast disintegrating properties are ensured by the presence of disintegration enhancing agents with the property of generating effervescence.

Specific examples of suitable disintegrating enhancing excipients include acid excipients, chosen from organic acids, acidic anhydrides, acid salts and carbonates.

It is clear that the combination of the aforementioned acidic excipients with carbonates generates effervescence which, when the cores enter in contact with water, exerts a burst effect promoting the immediate drug release from the core overcoming any possible matrix effect induced by residual quantities of cellulose ethers.

However, even though the final results are promising, this solution requires that the tableting process is being made at very low humidity levels to avoid earlier disintegration phenomena of the cores. Further, cores containing effervescent mixtures are typically coated by organic solvent-based systems to avoid the occurrence of gas formation on the surface of the coating cores when in contact with water used for aqueous-based coatings.

Moreover, large amounts of acidic excipients and carbonates are necessary to generate the effervescence needed to ensure the burst release of the drug. Hence, cores become large in weight and size thus requiring a longer coating phase reducing the process productivity.

To overcome the limitations of US 5,788,987, formulators could replace the disintegrating enhancing excipients capable of generating effervescence, with known disintegrants. Coating can be made by any suitable technique such as spray coating and powder layering.

However, when the outer coating is layered onto the cores by spraying an aqueous solution of hydrophilic ethers of cellulose, some cracking phenomena in the delaying-coating layer were observed with considerable consequences in the delay release characteristics of the finished product. Cracking phenomena were caused by the rapid swelling of the core induced when the disintegrant entered in contacts with an excess of aqueous solution during the coating processes due to insufficient drying conditions. More precisely, in the case of the spray coating technologies, water derived from the coating solution in which the hydrophilic polymers were solubilized, whereas in the case of the powder layering, it came from the binding solution used to enhance the adhesion of hydrophilic polymers in powdery form to the surface of the cores. In particular during the initial stages of the powder layering processes, the cores surface is likely to be over-wetted by the liquid binder spraying in order to onset the adhesion of the powder and the subsequent layering process. The alteration of the tablet cores surface was found to compromise the uniformity of the following layered coatings in terms of thickness, density and accordingly in terms of delaying efficiency characteristics.

To reduce the likelihood of cracking formation, one should reduce either the spraying rate of the coating solution (spray coating process) or of the binding solution (powder layering process) resulting in a process lengthening, or increasing the processing temperature introducing the risk of drug decomposition. Alternatively it is possible to utilize organic solvents alone or in mixture with water, with disadvantages over cost, safety and environmental pollution.

There is therefore the need to develop a new delayed/pulsatile release drug delivery system that encompasses the present state of the art.

In particular, the new presentations should ensure the manufacturing of pH independent delayed release cores containing one or more drugs; such cores after being coated with one or more hydrophilic polymers, are still capable of ensuring a fast drug release once the coating is eroded or dissolved by aqueous media; wherein the coating is preferably aqueous based, the hydrophilic polymers are made by one or more ether of cellulose, and the cores contain one or more disintegrating agents. In particular, the rapidly disintegrating cores should possess suitable mechanical resistance to withstand an aqueous based coating process.

Although the coating process used is preferably aqueous based, organic solvents may be used in any combination with water to shorten the processing time.

The object of the present invention has been reached by coating the rapidly disintegrating cores with two sequential layers. The inner layer, hereinafter defined as undercoat, seals the cores, whereas the outer layer exercises the delay release function.

Sealing can be performed by spraying techniques using aqueous or organic solvents or mixtures thereof. Aqueous based systems are preferred. Both the inner and outer layers are made by pH independent polymers. The undercoat is designed to prevent any premature swelling of the disintegrant in the core during the layering of the functional coating that ensures the desired delay release.

The undercoat does not modify the immediate release properties of the cores once the external layer is dissolved and/or eroded by aqueous media.

### DESCRIPTION OF THE INVENTION

Among the active principles that can be conveniently delivered by the novel time-specific delayed/pulsatile formulation are the short acting hypnotics for the treatment of sleep disorders. These active principles are capable of inducing hypnotic, sedative, anxiolytic, myorelaxant and anticonvulsive effects and may be used in prolonging the total sleep time or decreased number of awakening episodes.

Examples of such compounds include pyrazolopyrimidines, cyclopyrrolones, imidazopyridines, benzodiazepines, phenothiazines.

In particular, among the hypnotic active principles, Zaleplon a pyrazolopyrimidine compound, because of being rapidly absorbed with a time to peak concentration (t max) of approximately 1 hour, and rapidly eliminated with terminal-phase elimination half-life (t ½) of approximately 1 hour, can be considered a model candidate to be delivered with the oral time-specific delayed/pulsatile release formulations according to the present invention.

Because of its pharmacokinetic, Zaleplon, when delivered by an oral immediate release formulation taken at bed time, does not reach therapeutically peak plasma level during the early morning hours when the symptoms of early awakenings normally occur.

As a direct consequence, the molecule neither increases total sleep time, nor decreases the number of awakening. On the contrary, Zaleplon proved to be effective in shortening the time to sleep onset (TSO), suggesting the potential for use of the molecule for treating difficulties in initiating or maintaining sleep (Elie R., Zaleplon is effective in reducing time to sleep onset, European Neuropsychopharmacology, Volume 9, Supplement 5, September 1999, pp. 361-361(1)).

Accordingly, there is the need to include the active principle in a time-specific delayed release oral formulation which, taken at bed time, is capable of ensuring therapeutically effective peak plasma level to treat sleep disorders during the early morning hours. Moreover, to avoid the risk of inducing any hangover during the awakening hours, it is vital that the Zaleplon is rapidly released once the programmed delay time is elapsed. This achievement can be obtained thanks to the rapid disintegration properties ensured by the cores of the present invention once the external functional hydrophilic polymeric layer is dissolved and /or eroded by the body fluids.

However, also other active principles can be delivered conveniently according to the teaching of the present invention. A non limitiative list includes, amino acids, peptides, enzymes, hormones, anti-infective agents, anticonvulsivants, central nervous system stimulants, cholinergic and anticholinergic agents, anti-parkinsonians, antihistaminics β₂₋ adrenergic receptor agonists, anti-asthmatics, anti-inflammatory analgesics, cardiovascular agents.

### Core manufacturing

The cores of the time-specific formulations can be in the form of tablets or minitablets (i.e. cylindrical tablets with diameter in the range of 1.5 - 3.0 mm) or pellets (core containing spheroids with diameter 300 - 2000µm). Each core contains, in addition to one or more active principles, at least one disintegrant and known tableting (and for pellets, pellettizing) adjuvants such as but not limited to soluble or insoluble fillers, binding agents, glidants, anticaking agents, buffering agents, preservatives, antioxidants, surfactants, chelating agents, lubricants, etc.

Disintegrating agents suitable to be used in the present invention can be chosen from different classes, or mixtures thereof, here below summarised.

Modified celluloses such as cross-linked sodium carboxymethylcellulose, cross-linked polyvinylpyrrolidone such as crospovidone, natural starches such as maize starch, potato starch, directly compressible starches such as starch 1500, modified starches such as carboxymethylstarches and sodium starch glycolate, starch derivatives such as amylose, alginic acid and sodium alginate, microcrystalline cellulose.

Cross-linked sodium carboxymethylcellulose and crospovidone are the disintegrant preferred.

Typically once the uncoated core is put in a glass of water, its complete disintegration occurs within 5 minutes. The disintegration properties may also be conveniently modified by the presence of soluble and insoluble fillers and by their weight ratio thereof.

The insoluble excipients can be selected from the group of microcrystalline cellulose, calcium phosphate tribasic, dibasic calcium phosphate, calcium sulphate and dicalcium phosphate. Dicalcium phosphate either anhydrous or hydrated is preferred.

The soluble excipients can be selected from the group of lactose, sorbitol, xylitol, mannitol, amylose, dextrose, fumaric acid, citric acid, tartaric acid, lactic acid, malic acid, ascorbic acid, succinic acid, polyethylene glycols of various molecular weight, soluble hydroxyalkylcelluloses, polyvinylpyrrolidones, gelatins, sodium carbonate, sodium bicarbonate, sucrose .

With respect to the weight of the uncoated cores, the at least one active principle is present in amounts of about 1-80%, preferably 5-50% and the at least one disintegrant is amounts of 0,5 - 20%, preferably 1 - 10%.

Depending on the rheological properties of the mixture to be tabletted, cores can be prepared by any known technique such as direct compression, dry granulation, wet granulation, melt granulation.

Pellet cores prepared by the same formulation described for tablets can be obtained by any known technique such as extrusion-spheronization, direct pelletization, drug layering.

In another embodiment of the invention, cores may be a multi-layer tablet designed to ensure a pulsatile drug release. This approach is thought for drugs that need to be delivered with different dissolution kinetics in a single daily dose once the programmed lag time is elapsed.

In the case of a bi-layered tablet, this target can be achieved by fractioning the dose into two parts i.e., the immediate release fraction in a layer comprising the disintegrant, the modified release fraction in a layer comprising excipients that exert the release control. Alternatively different active principles can be included each in one separate layer of the tablet.

Among the excipients exerting sustained release (in particular in the case of the multi-layer tablet) are polymers belonging to the alkylcelluloses such as hydroxypropyl methylcellulose, hydroxypropylcellulose, hydroxyethylcellulose, methylcellulose, carboxymethylcellulose, and polymers selected from the group of polyvinylpyrrolidone, copovidone, polyethylene glycols, polyvinylalcohol-polyethylene glycol copolymer, polyvinyl acetate, poly(ethylacrylate, methyl methacrylate) 2:1, poly(ethyl acrylate, methyl methacrylate, trymethylammonioethyl methacrylate chloride) 1:2:0.2, poly(ethyl acrylate, methyl methacrylate, trymethylammonioethyl methacrylate chloride) 1:2:0.1., cross-linked polyacrylic acid derivatives, natural gums such as xanthan gum

Alternatively the release control may be ensured by wax like excipients alone or in combination with the aforementioned polymers.

A not limitative list of suitable excipients includes stearates, glyceryl esters, waxes (carnauba, cethyl esters, microcrystalline), alone or mixtures thererof.

This embodiment is effective to deliver also cardiovascular agents, such as but not limited to the angiotensin converting enzyme (ACE) inhibitors, and anti-inflammatory analgesics. Among the ACE inhibitors, Ramipril, because of being rapidly absorbed and eliminated, can be considered a model candidate in the antihypertensive area when a single daily dose administration is required to cover the 24h. In such a case, the pulsatile formulation taken at bed time is capable of ensuring a burst drug release during the early morning hours (i.e. when the pressure levels reach the maximum intensity) associated to a slow drug release that contributes to the maintenance of the pressure control along the day.

For the same pharmacokinetic reasons, among the anti-inflammatory analgesics, Lornoxicam, an oxicam derivative, can be conveniently delivered by a bi-layer tablet capable of releasing the active principle with two different dissolution kinetics (i.e. immediate release and modified release) in a single daily dose.

### Core sealing (undercoat)

The undercoat essentially consists of one or more pH independent water soluble and/or water insoluble polymers. This means that those polymers are the main constituents of the undercoat which, nevertheless, may further contain minor amounts of excipients or adjuvants whose content, however, does not exceed 20% by weight, preferably 10%, of the undercoat itself. Those polymers are layered onto the cores by spraying, in a coating pan or in a fluid bed, a polymeric solution or dispersion, using aqueous or organic solvents or mixture thereof.

Preferably the undercoat is layered in an aqueous environment.

The polymers are selected from the group of polyvinylpyrrolidone, copovidone, polyethylene glycols, polyvinylalcohol-polyethylene glycol copolymer, polyvinyl acetate, poly(ethylacrylate, methyl methacrylate) 2:1, poly(ethyl acrylate, methyl methacrylate, trymethylammonioethyl methacrylate chloride) 1:2:0.2, poly(ethyl acrylate, methyl methacrylate, trymethylammonioethyl methacrylate chloride) 1:2:0.1, ethers of cellulose (alkylcelluloses) such as hydroxypropylmethylcellulose, hydroxylpropylcellulose, hydroxyethylcellulose, methylcellulose, ethylcellulose, cellulose acetate, carboxymethyl cellulose, their derivatives and mixtures thereof.

Alkylcelluloses having low molecular weight are the polymers preferred. These ethers of cellulose are commercialised in a number of different grades with different apparent viscosities and degree of substitution The cellulose ether has an apparent viscosity varying in the range of 2 cP to 100 cP (2% aqueous solution, 20°C), preferably from 2 to 45 cP, even more preferably from 2 to 20 cP. The preferred ethers of cellulose are hydroxypropyl methylcelluloses with a degree of substitution (%) ranging between 19-30, preferably 28-30[DP1] (methoxyl group) and 7- 12 (hydroxypropyl group).

Additional functional coating excipients such as anti-sticking agents, plasticizers, waxes, surfactants, pigments, pore formers, pH adjusters, buffering agents etc, may be part of the polymeric film.

Typically the undercoat is layered to achieve a weight gain of the starting cores between 0.1 and 10%, preferably between 0.5 to 5% as determined by solid substance. For example, given uncoated cores each weighing 100 mg, an undercoat of 5% expressed as weigh gain, means that the sealed cores reach a weight of 105 mg each. The undercoat is designed to let unmodified the disintegration characteristics of the uncoated cores.

### Delayed Release Coating

Sealed cores are coated with a polymeric film comprising one or more pH independent, hydrophilic, polymers. After the intake, the polymeric coating hydrates to form a gel-like layer that delays drug release from the cores until it is completely or partially dissolved and/or eroded by the body fluids. Drug release takes places after a pre-defined period of time depending on the coating thickness achieved and polymer mixture composition.

This functional coating delays the drug release from the cores for the programmed period of time depending on the thickness of the coating layer.

The expression "the outer coating, surrounding the sealing layer, essentially consisting of at least one hydrophilic polymer" means that said one or more polymers are the main constituents of the outer coating which, nevertheless, may further contain minor amounts of excipients or adjuvants whose content, however, does not exceed 20% by weight, preferably 10%, of the outer coating itself.

Coating is performed by spraying, in a coating pan or in a fluid bed, the cores with a polymeric solution or dispersion, using aqueous or organic solvents or mixture thereof. Preferably the undercoat is layered in an aqueous environment.

Alternatively the coating may also be layered in powdery form by spraying the cores with a binder liquid and simultaneously or alternatively spreading them with a mixture in powdery form comprising one or more pH independent, hydrophilic polymers.

Suitable binding solution may include pharmaceutically acceptable binding agents solubilized in a suitable solvent. Even though water is the preferred solvent, other examples of suitable solvents either aqueous or organic or mixture thereof will be appreciated by those skilled in the art and are contemplated by the methods of the present invention.

Examples of binding agents include but are not limited to vinyl polymers such as polyvinylpyrrolidone, polyvinyl alcohol, and the like, cellulosic polymers, such ashydroxypropylcellulose, hydroxyethylcellulose, hydroxypropylcellulose, and the like, acrylic polymers and copolymers such as methacrylic acid copolymers, ethyl acrylate-methylmethacrylate copolymers and the like, natural or synthetic gums such as guar gum, arabic gum, xanthan gum, and the like, gelatine, pectin; and mixture thereof. Polyvinylpyrrolidone and hydroxypropyl methylcellulose are the preferred binders.

Among the polymers of choice constituting the delayed release coating are the alkylcelluloses (such as hydroxypropyl methylcellulose, hydroxypropylcellulose, hydroxyethylcellulose, methylcellulose, carboxymethylcellulose) and the polyethylene glycols. Additional functional coating excipients such as anti-sticking agents, glidants, plasticizers, waxes, surfactants, pigments, pore formers, pH adjusters, buffering agents etc, may be part of the functional polymeric film coating.

Hydroxypropyl methylcelluloses are the preferred alkylcellulose polymers.

In one preferred embodiment they possess, alone or in a blend, a nominal viscosity in the range of 5 cp to 4,000 cP, preferably in the range of 46 to 400 cP (2% water solution, 20°C), and a degree of substitution % ranging between 19-30, preferably 28-30 (methoxyl group) and 7-12 (hydroxypropyl group).

In another preferred embodiment, the coating comprises one or more alkylcelluloses in combination with polyethylene glycol, in weight ratio between 20:1 and 1: 5, preferably between 15:1 and 1:1, where the polyethylene glycol has a molecular weight in the range of approximately 200 to 9000, preferably of approximately 400 to 6000.

Typically the delayed release coating level expressed as weight gain % may vary in the range between 5 and 500, preferably between 10 and 200% as determined by solid substance. For example, given sealed cores each weighing 105 mg, an undercoat of 60% expressed as weigh gain, means that the coated cores reach a weight of 168 mg each.

The invention is further illustrated by the following non limitative examples.

### EXAMPLE 1 (comparative example)

### Core manufacturing

A batch of 4 Kg of Zaleplon 10 mg uncoated tablets (lot P-06-031) was produced by direct compression.

The quali-quantitative formula is shown on table I.

**Tab. I**

| **Ingredients** | **Quantity (mg /tablet)** |
|---|---|
| 1. Zaleplon | 10.0 |
| 2. Dicalcium phosphate anhydrous | 34.0 |
| 3. Lactose monohydrate | 23.0 |
| 4. Sodium lauryl sulphate | 1.0 |
| 5. Cellulose microcristalline | 42.4 |
| 6. Carboxymethylcellulose XL | 4.5 |
| 7. Silicon dioxide | 0.5 |
| 8. Mg Stearate | 0.6 |
| **Total** | **116.0** |

All the ingredients were sift trough a 710 µm sieve. Components 1 to 7 were mixed in a 12 L cube blender for 25 minutes at 15 rpm, than component no. 8 was added and the mixture further rotated for 5 minutes. The final mixture was compressed at 9 KN with a rotary tableting machine using round convex punches having a diameter of 6 mm and a curvature radius of 6 mm. At the end of the process 3.6 Kg of tablets weighing each 116 mg were obtained.

The main physical-technological characteristics are reported on table II.

**Tab II**

| **Test** | **Results** |
|---|---|
| Average weight | 116 mg |
| Hardness (EP 5^{th} ed.) | 120N |
| Friability (EP 5^{th} ed.) | 0.05% |
| Disintegration time (EP 5^{th} ed.) | < 1 min |

The uncoated tablets lot P-06-031 were subjected to in vitro dissolution analysis (UV = 232 nm).

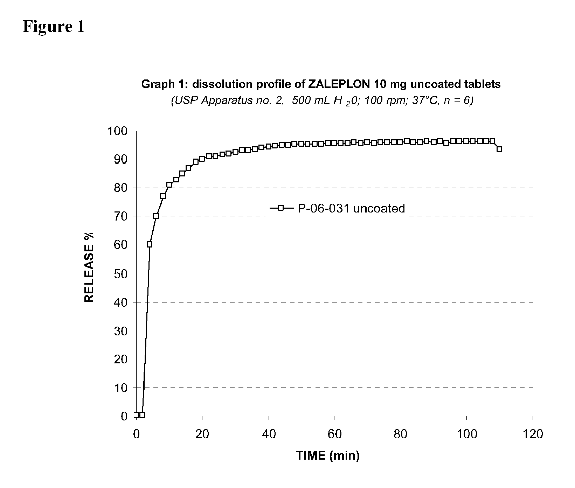

The results are shown in figure 1, which shows an immediate and sharp increase of the dissolution curve indicating that the tablets once in contact with aqueous media immediately disintegrated promptly releasing the active.

### Delayed Release Coating

1.8 Kg of the uncoated cores lot P-06-031 were loaded into a side-vented coating pan and sprayed with a 6.6% w/w aqueous solution of hydroxypropyl methylcellulose type 2910, 50cp and polyethylene glycol type 400, in a weight ratio of 10:1. Coating continued until a weigh gain of 50% of total tablet weigh was achieved corresponding to a tablet weight of 174 mg. In Table III processing conditions are reported.

**Tab III**

| **Processing parameters** | **Dalayed release coating** |
|---|---|
| Inlet air temperature (°C) | 65 |
| Outlet air temperature (°C) | 47 |
| Cores temperature (°C) | 46 |
| Rotating pan speed (rpm) | 24 |
| Inlet air volume (m³/h) | 286 |
| Coating system spraying rate (g/min) | 16 |
| Atomizing pressure (bar) | 2.1 |

At the end of the process, observing the coated product, a considerable number of tablets showed cracking lines affecting the coating surface that might have an impact on the dissolution kinetic.

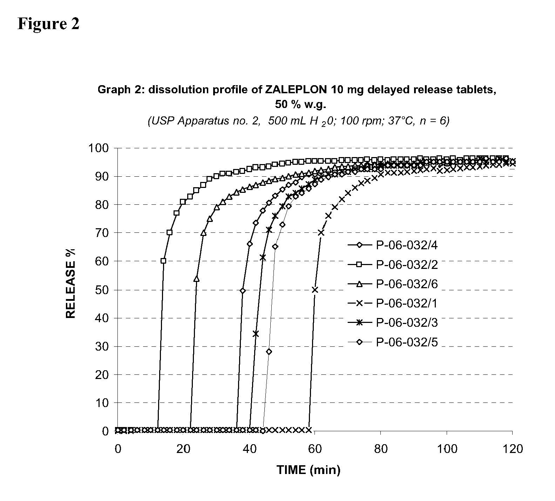

The coated tablets lot P-06-032 were subjected to in vitro dissolution analysis (UV = 232 nm). The results are summarised in figure 2.

Figure 2 shows a broad variation of dissolution profiles from tablet to tablet. Drug release onset took place in the range of approximately 15 minutes (vessel # 2) to approximately 60 minutes (vessel # 1). It was evident that the cracking lines observed onto the coating surfaces severely altered the delayed release properties induced by the coating layer.

### EXAMPLE 2

### Core Sealing

The remaining 1.8 Kg of uncoated Zaleplon 10 mg tablets lot P-06-031 (see example 1 for the manufacturing details) were loaded into a side-vented coating pan and sprayed with a with a 6.6% w/w aqueous solution of hydroxypropyl methylcellulose type 2910, 5cp and polyethylene glycol type 400, in a weight ratio of 10:1. Coating continued until a weigh gain of 3% of total tablet weigh was achieved corresponding to a tablet weight of 119.5 mg. Coating conditions are reported in Table IV.

### Delayed Release Coating

The sealed cores were than coated using the same apparatus used for the sealing coating with a 6.6% w/w aqueous solution of hydroxypropylmethylcellulose type 2910, 50cp and polyethylene glycol type 400, in a weight ratio of 10:1. Coating continued until a weigh gain of 50% of total tablet weigh was achieved corresponding to a tablet weight of 179.2 mg. Coating conditions are reported in Table IV.

**Tab IV**

| **Processing parameters** | **Cores sealing** | **Dalayed release coating** |
|---|---|---|
| Inlet air temperature (°C) | 57 | 65 |
| Outlet air temperature (°C) | 45 | 47 |
| Cores temperature (°C) | 40 | 46 |
| Rotating pan speed (rpm) | 16 | 24 |
| Inlet air volume (m³/h) | 266 | 286 |
| Coating system spraying rate (g/min) | 10 | 16 |
| Atomizing pressure (bar) | 2,1 | 2,1 |

At the end of the process, no coating alterations were noticed. The coated tablets lot P-06-033 were subjected to in vitro dissolution (UV = 232nm).

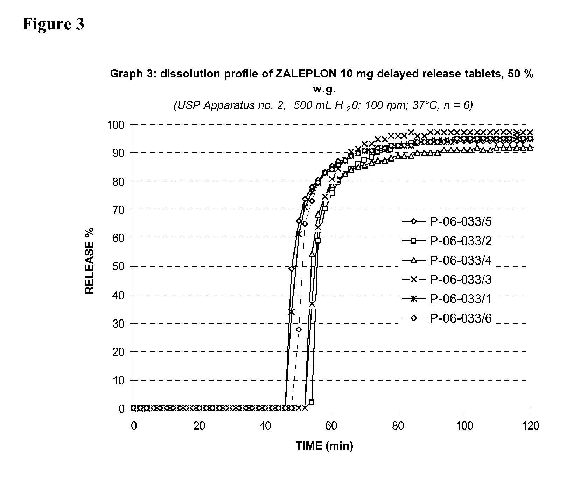

The results are summarised in figure 3, which shows that all the six tablets produced according to the present invention are characterized by a narrow variation of the dissolution profiles associated to a rapid dissolution kinetic that starts, for all the units analysed, approximately in the range 45- 55 min.

Moreover, once the system initiates the drug release, no matrix effect can be observed as the dissolution kinetic of the delayed release tablets appears to be still superimposable with the corresponding uncoated (see figure 1).

### EXAMPLE 3

### Core manufacturing

25 Kg of Zaleplon 10 mg uncoated tablets lot P-06-034 were produced by direct compression using the same composition displayed in Table I.

All the ingredients were sift trough a 710 µm sieve. Components 1. to 7. were mixed in a 90 L tumbler blender for 25 minutes at 10 rpm, than component no.8 was added and the mixture further rotated for 5 minutes. The final mixture was compressed at 9 KN with a rotary tableting machine using round convex punches having a diameter of 6 mm and a curvature radius of 6 mm. At the end of the process 22 Kg of tablets weighing each 115 mg were obtained.

The main physical-technological characteristics are reported on table V.

**Tab V**

| **Test** | **Results** |
|---|---|
| Average weight | 115 mg |
| Hardness (EP 5^{th} ed.) | 122N |
| Friability (EP 5^{th} ed.) | 0.05% |
| Disintegration time (EP 5^{th} ed.) | < 1 min |

The uncoated tablets lot P-06-034 were subjected to in vitro dissolution analysis (UV = 232 nm).

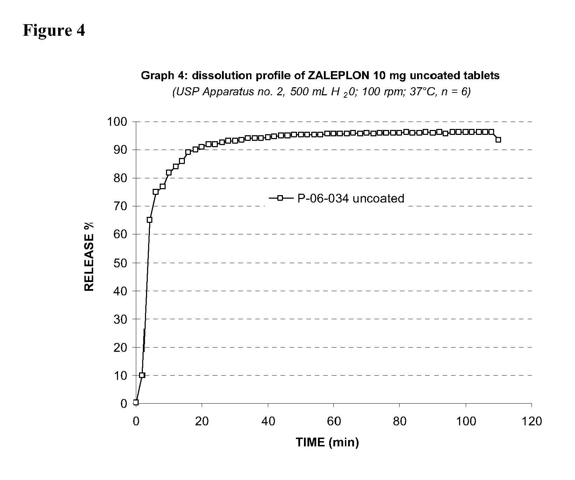

The results are shown in figure 4, which shows an immediate and sharp incline of the dissolution curve indicating that the tablets once in contact with aqueous media immediately disintegrated promptly releasing the active.

### Delayed Release Coating

10Kg of tablets of the batch P-06-034 were coated by a powder layering technique spreading a powder mixture containing hydroxypropyl methylcellulose type 2910, 50cp (94.0%), talc (4.5%) and silicon dioxide (1.5%) and alternatively spraying a 6.6% w/w aqueous solution of hydroxypropyl methylcellulose type 2910, 50cp and polyethylene glycol type 400, in a weight ratio of 10:1, as binder solution. Coating process continued until a weigh gain of 50% of total tablet weigh was achieved corresponding to a tablet weight of 180.0 mg. Processing condition relevant to delayed release coating are summarized in Table VI.

**Tab. VI**

| **Processing parameters** | **Delayed release coating** |
|---|---|
| Rotating pan speed (rpm) | 22 |
| Atomizing air pressure (bar) | 1,0 |
| Liquid spray rate (g/min) | 50 |
| Powder feeling rate (g/min) | 75 |
| Inlet air temperature (°C) | 75 |
| Outlet air temperature (°C) | 29 |
| Product temperature (°C) | 25 |
| Inlet air volume (m³/h) | 100 |

At the end of the process, evident volume alterations of the layered coating were noticed. The coated tablets lot P-06-035 were subjected to in vitro dissolution (UV = 232nm).

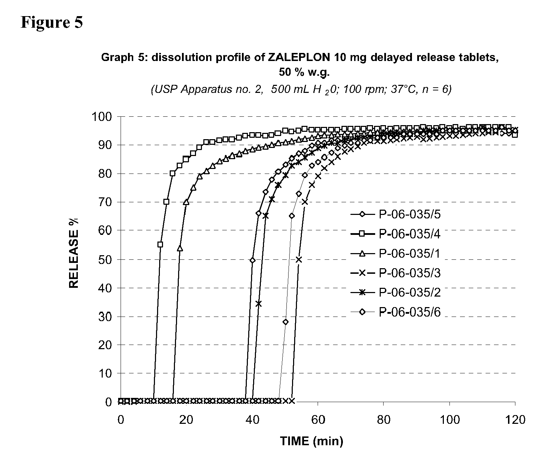

The results are summarised in figure 5, shows a broad variation of dissolution profiles from tablet to tablet. Drug release onset took place in the range of approximately 15 minutes (vessel # 4) to approximately 54 minutes (vessel # 3).

It was evident that the cracking lines observed onto the coating surfaces severely altered the delayed release properties induced by the coating layer.

### EXAMPLE 4

### Core Sealing

A 10Kg batch of uncoated Zaleplon 10 mg tablets from lot P-06-034 was loaded into a conventional coating pan and sprayed with a with a 6.6% w/w aqueous solution of hydroxypropyl methylcellulose type 2910, 5cp and polyethylene glycol type 400, in a weight ratio of 10:1. Coating continued until a weigh gain of 3% of total tablet weigh was achieved corresponding to a tablet weight of 118.5 mg.

### Delayed Release Coating

The sealed cores were than coated by a powder layering technique spreading a powder mixture containing hydroxypropyl methylcellulose type 2910, 50cp (94.0%), talc (4.5%) and silicon dioxide (1.5%) and alternatively spraying a 6.6% w/w aqueous solution of hydroxypropyl methylcellulose type 2910, 50cp and polyethylene glycol type 400, in a weight ratio of 10:1, as binder solution. Coating continued until a weigh gain of 50% of total tablet weigh was achieved, corresponding to a tablet weight of about 180 mg.

**Tab VII**

| **Processing parameters** | **Cores sealing** | **Delayed release coating** |
|---|---|---|
| Rotating pan speed (rpm) | 20 | 22 |
| Atomizing air pressure (bar) | 2.1 | 1.0 |
| Liquid spray rate (g/min) | 18 | 50 |
| Powder feeling rate (g/min) | 0 | 75 |
| Inlet air temperature (°C) | 70 | 75 |
| Outlet air temperature (°C) | 50 | 29 |
| Product temperature (°C) | 25 | 25 |
| Inlet air volume (m³/h) | 200 | 100 |

At the end of the process, no coating alterations were noticed. The coated tablets lot P-06-036, were subjected to in vitro dissolution (UV = 232nm).

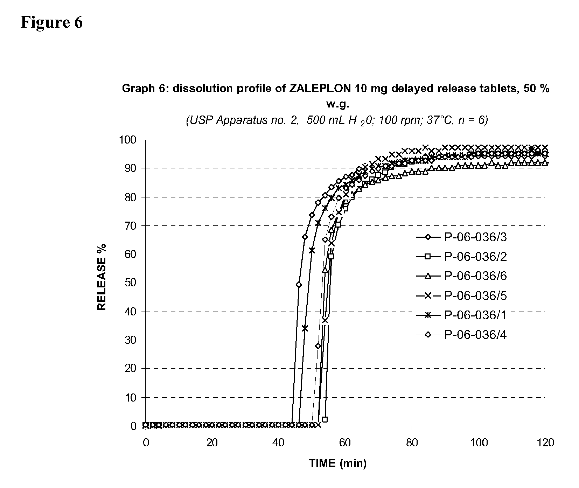

The results are summarised in figure 6, which shows that all the six tablets produced according to the present invention are characterized by a narrow variation of the dissolution profiles associated to a rapid dissolution kinetic that starts, for each of the units analysed, approximately in the range 45- 55 min.

Moreover, once the system initiates the drug release, no matrix effect can be observed as the dissolution kinetic of the delayed release tablets appears to be still superimposable with the corresponding uncoated (see figure 4).

## Claims

1. A pharmaceutical formulation which comprises:
• a core comprising at least one active principle and at least one disintegrating agent;
• a sealing layer, surrounding the core, essentially consisting of at least one polymer;
• an outer coating, surrounding the sealing layer, essentially consisting of at least one hydrophilic polymer;
wherein:
the at least one disintegrating agent is present in amounts of 0.5-20% by weight and the at least one active principle is present in amounts of 1-80% by weight, with respect to the core;
the sealing layer accounts for 0.1-10% by weight, with respect to the core;
the outer coating accounts for 5-500 % by weight, with respect to the core.

2. A pharmaceutical formulation according to claim 1, **characterized in that** the water solubility of the polymers contained in the sealing layer and in the outer coating is pH independent.

3. A pharmaceutical formulation according to claim 1, **characterized in that** the at least one polymer contained in the sealing layer is other than the at least one polymer contained in the outer coating.

4. A pharmaceutical formulation according to claim 1, **characterized in that** the at least one disintegrating agent is present in amounts of 1-10% by weight, with respect to the core.

5. A pharmaceutical formulation according to claim 1, **characterized in that** the at least one active principle is present in amounts of 5-500% by weight, with respect to the core.

6. A pharmaceutical formulation according to claim 1, **characterized in that** the sealing layer accounts for 0.5-5% by weight, with respect to the core.

7. A pharmaceutical formulation according to claim 1, **characterized in that** the outer coating accounts for 10-200 % by weight, with respect to the core.

8. A pharmaceutical formulation according to claim 1, **characterized in that** said at least one disintegrating agent is selected from modified cellulose, natural starch, directly [DP2]compressible starch, modified starch, starch derivatives and microcrystalline cellulose.

9. A pharmaceutical formulation according to claim 8, **characterized in that** said modified cellulose is selected from cross-linked sodium carboxymethylcellulose and cross-linked polyvinylpyrrolidone; said natural starch is selected from maize starch and potato starch; said directly compressible starch is starch 1500; said modified starch is selected from carboxymethylstarch and sodium starch glycolate; said starch derivative is selected from amylose, alginic acid and sodium alginate.

10. A pharmaceutical formulation according to claim 1, **characterized in that** said at least one active principle is selected from hypnotics, sedatives, anxiolytics, myorelaxants andanticonvulsives.

11. A pharmaceutical formulation according to claim 1, **characterized in that** said at least one active principle is selected from pyrazolopyrimidines, cyclopyrrolones, imidazopyridines, benzodiazepines and phenothiazines.

12. A pharmaceutical formulation according to claim 1, **characterized in that** said at least one active principle is Zaleplon.

13. A pharmaceutical formulation according to claim 1, **characterized in that** the core is a multi -layered tablet, preferably a bi-layered tablet.

14. A pharmaceutical formulation according to claim 1, **characterized in that** said at least one polymer of the sealing layer is selected from the group of polyvinylpyrrolidone, copovidone, polyethylene glycols, polyvinylalcohol-polyethylene glycol copolymer, polyvinyl acetate, poly(ethylacrylate, methyl methacrylate) 2:1, poly(ethyl acrylate, methyl methacrylate, trymethylammonioethyl methacrylate chloride) 1:2:0.2, poly(ethyl acrylate, methyl methacrylate, trymethylammonio ethyl methacrylate chloride) 1:2:0.1, ethers of cellulose (alkylcelluloses) such as hydroxypropylmethylcellulose, hydroxylpropylcellulose, hydroxyethylcellulose, methylcellulose, ethylcellulose, cellulose acetate, carboxymethyl cellulose.

15. A pharmaceutical formulation according to claim 1, **characterized in that** said at least one polymer of the sealing layer is an alkylcellulose whose 2% by weight aqueous solution at 20°C has an apparent viscosity of from 2 cP to 100 cP, preferably from 2 to 45 cP, even more preferably from 2 to 20 cP.

16. A pharmaceutical formulation according to claim 15, **characterized in that** said at least one alkylcellulose is hydroxypropylmethylcellulose.

17. A pharmaceutical formulation according to claim 1, **characterized in that** said at least one hydrophilic polymer of the outer coating is selected from alkylcelluloses and polyethylene glycols.

18. A pharmaceutical formulation according to claim 17, **characterized in that** said alkylcellulose is selected from hydroxypropylmethylcellulose, hydroxypropylcellulose, hydroxyethylcellulose, methylcellulose, carboxymethylcellulose.

19. A pharmaceutical formulation according to claim 17, **characterized in that** said polyethylene glycol has a molecular weight of from 200 to 9000 Da, preferably from 400 to 6000 Da.

20. A pharmaceutical formulation according to claim 1, **characterized in that** said at least one hydrophilic polymer of the outer coating is an hydroxypropylmethylcelluloses whose 2% by weight aqueous solution at 20°C has an apparent viscosity of from 5 cP to 4,000 cP, preferably from 46 cP to 400 cP.

21. A pharmaceutical formulation according to claim 1, **characterized in that** the outer coating essentially consists of one or more alkylcelluloses in combination with one or more polyethylene glycols, wherein the alkylcellulose: polyethylene glycol weight ratio is between 20:1 and 1:5, preferably between 15:1 and 1:1.
